# EUROPEAN PATENT SPECIFICATION

(11) **EP 2 540 328 B1**
(45) Date of publication and mention of the grant of the patent: **25.11.2015**
(21) Application number: 12173399.2
(22) Date of filing: 25.06.2012
(51) Int. Cl.: A61M 1/36

(54) **Device for filtering arterial blood**
Vorrichtung zum Filtern von arteriellem Blut
Dispositif pour filtrer le sang artériel

(30) Priority: 27.06.2011 IT MO20110161
(43) Date of publication of application: 02.01.2013
(73) Proprietor: Eurosets S.r.l., 41036 Medolla (MO) (IT)
(72) Inventor: Ghelli, Nicola, 40018 S. Pietro in Casale (BO) (IT); Balanzoni, Roberto, 41036 Medolla (MO) (IT); Costa, Edgardo, 41036 Medolla (MO) (IT); Zerbini, Alessandro, 41036 Medolla (MO) (IT)
(74) Representative: Zoli, Filippo

(56) References cited:
- EP-A1- 1 203 592
- GB-A- 2 183 168
- US-A1- 2004 228 760
- US-B1- 6 918 887

## Description

The present invention relates to a device for filtering arterial blood.

As is known, during the course of a number of surgical operations, an extracorporeal circulation becomes necessary of the patient's blood in a circuit which comprises a number of appliances suitable for ensuring the correct treatment of the blood itself.

Generally, a circuit for the extracorporeal circulation of blood comprises the following devices which are interlinked by means of related connection lines: a tank for containing the blood flowing from the patient, also called venous blood, a pump for conveying the blood in the circuit, a heat exchanger suitable for ensuring the correct blood temperature, an oxygenator with the function of ceding oxygen to the blood and a filter suitable for treating the blood flowing from the oxygenator, also called arterial blood, to trap any air bubbles in the blood itself.

The present treatise relates in particular to the device for filtering arterial blood which is arranged downstream of the oxygenator.

The filtering devices for filtering arterial blood of known type generally comprise a body defining a filtering chamber inside which is arranged an internally hollow filtering element.

More in detail, the filtering chamber has an inlet port for the blood to be filtered, an outlet port for the filtered blood and an air discharge port.

The filtering element, which defines a closed profile, divides the filtering chamber into a chamber inside and a chamber outside the filtering element itself where the outer chamber is arranged around the inner chamber.

Generally, the inlet port is positioned in the upper part of the body facing the outer chamber, which is therefore suitable for containing the blood to be filtered, while the inner chamber is suitable for containing the filtered blood. The blood flowing from the oxygenator is introduced inside the outer chamber, where it makes a substantially helical trajectory around the filtering element, is pushed in a radial direction through the filtering element itself, is collected in the inner chamber and exits towards the outside of the body. During the filtering stage, the air bubbles in the blood to be filtered remain in the outer chamber and naturally convene upwards, where the related discharge port is positioned. These known filtering devices do have a number of drawbacks however.

In fact, following the introduction of the blood to be filtered into the outer chamber, pockets of air can form in correspondence to the bottom of the outer chamber itself. These pockets of air must be suitably removed before subsequent use, and this operation, called "air removal", calls for the filtering device to be overturned and shaken manually. Furthermore, because the outer surface of the filtering element is generally folded to form ribs (also called pleats), it can occur that a certain quantity of air bubbles remain trapped between such ribs and, consequently, in this case as well, it is best to remove them manually between one use and another.

These known devices are not therefore very easy to use for health operators, who must dedicate time to remove the filtering device from the relative connections and proceed with the air removal operation.

Furthermore, the need to move the above-described filtering devices to perform air removal also makes their integration with the oxygenator complicated, i.e., the realization of an oxygenator and a filtering device integrally associated with one another.

Another drawback of the known filtering devices consists in the fact that the air bubbles are pushed by the centrifugal force towards the filtering element, with the risk therefore of their passing through this, further breaking up and ending up in the arterial line leading to the patient.

EP 1 203 592 discloses a filtering device comprising a box body inside which is placed an hollow filtering element that delimits the internal chamber of the box body into an inner and an outer chamber.

The main object of the present invention is to provide a device for filtering arterial blood which is able to considerably reduce the presence of air remaining inside it during filling and which is therefore easier and more practical to use compared to devices of known type.

Within this aim, one object of the present invention is therefore to provide a filtering device that does not require any manual intervention t2ao eliminate any air remaining inside it.

Another object of the present invention is to provide a filtering device which allows obtaining a more efficient separation of the air from the blood compared to devices of known type and which therefore reduces the risk of the presence of air bubbles in the arterial blood which is infused to the patient.

Yet another object of the present invention is to provide a device that can be easily integrated with an oxygenator.

Another object of the present invention is to provide a device for filtering arterial blood which allows to overcome the mentioned drawbacks of the state of the art within the ambit of a simple, rational, easy and effective to use as well as low cost solution.

The above objects are achieved by the present device for filtering arterial blood as defined in the appended claims.

Other characteristics and advantages of the present invention will become more evident from the description of a preferred, but not sole, embodiment of a device for filtering arterial blood, illustrated purely as an example but not limited to the annexed drawings in which:
figure 1 is a section view of an embodiment of the device according to the invention;
figure 2 is a section view of an alternative embodiment of the device according to the invention;
figure 3 is a section view of an alternative embodiment of the device according to the invention.

With particular reference to such figures, globally indicated by 1 is a device for filtering arterial blood.

The device 1 comprises a body 2 which defines a filtering chamber 3, 4 having at least an inlet port 5 of the blood to be filtered, at least an outlet port 6 of the filtered blood and at least a discharge port 7 of the air to the outside. The filtering chamber 3, 4 is therefore that inside which the blood coming from the inlet port 5 is filtered.

In the present treatise, the terms inlet port, outlet port and discharge port are used to identify the ports, defined by the body 2, directly facing onto the filtering chamber 3,4. Such ports do not necessarily coincide with the openings of the body 2 which face onto the outside. More in particular, the body 2 can have one or more ducts communicating on the one side with the outside and on the opposite side with one of either the inlet port, the outlet port or the discharge port.

Preferably, the body 2 is substantially bell shaped and the discharge port 7 is defined in correspondence to the upper portion of the filtering chamber 3, 4. Advantageously, at least one between either the inlet port 5 and the outlet port 6 is arranged in correspondence to the lower portion of the filtering chamber 3, 4. Preferably, both the inlet port 5 and the outlet port 6 are arranged in correspondence to the lower portion of the filtering chamber 3,4.

In the preferred embodiments shown in the illustrations, the inlet port 5 is defined in correspondence to the base of the body 2, the outlet port 6 is defined in correspondence to the lateral surface of the body 2 and the discharge port is defined in correspondence to the upper top of the body 2.

The terms "lower" and "upper" used in the present treatise refer to the position taken up by the device in normal operating conditions.

The device 1 then comprises at least one filtering element 8 hollow on the inside and arranged at least partially inside the body 2.

The filtering element 8 is therefore housed at least in part inside the filtering chamber 3,4 and delimits an inner chamber 3 and an outer chamber 4 with respect to the filtering element itself, where the outer chamber 4 surrounds the inner chamber 3 at least in part.

The filtering chamber 3,4 can therefore completely or in part contain the filtering element 8.

More in particular, the inner chamber 3 is delimited laterally by the filtering element 8 only while the outer chamber 4 is substantially positioned between the filtering element 8 and the body 2.

The filtering surface is therefore that which delimits the chambers 3 and 4 laterally.

Advantageously, the filtering element 8 has a substantially circular cross section. In the preferred embodiments shown in the illustrations, the filtering element 8 has a substantially tubular shape and therefore has a lateral surface 8a, defining the filtering surface, and two bases 8b and 8c opposite one another, of which one lower base 8b and one upper base 8c.

Preferably, the filtering surface 8a is contained inside the filtering chamber 3,4. According to the invention, the inner chamber 3 and the outer chamber 4 are suitable for containing the blood to be filtered and the filtered blood respectively and are therefore in communication with the inlet port 5 of the blood to be filtered and with the outlet port 6 of the filtered blood respectively. The inner chamber 3 and the outer chamber 4 therefore correspond to the blood pre-filtering chamber and to the blood post-filtering chamber respectively. According to the invention, furthermore, the inner chamber 3 has an inlet mouth 3a for the blood to be filtered defmed in correspondence to the lower base 8b of the filtering element 8.

In use, the blood to be filtered flows through the inlet mouth 3a to enter the inner chamber 3, where the air bubbles naturally collect up in the upper part of the inner chamber itself by effect of their lower weight, passes through the filtering element 8 and collects up in the outer chamber 4 before exiting from the outlet port 6. The path followed by the blood and the air inside the device 1 is indicated by the arrows 9 and 19, shown in the illustrations, respectively. Advantageously, the body 2 comprises a transit channel 10 for the blood to be filtered, communicating on the one side with the inlet port 5 and on the opposite side with the outside, suitable for introducing the blood to be filtered into the inner chamber 3 through the inlet mouth 3a thereof, where the latter is arranged in correspondence to the inlet port 5.

Preferably, the channel 10 is suitable for introducing the blood to be filtered in correspondence to the median area of the inlet mouth 3a, i.e. substantially along the longitudinal axis of the inner chamber 3.

Alternative embodiments cannot however be ruled out wherein the blood to be filtered is introduced tangentially into the inlet mouth 3a, so as to create a whirling movement inside the inner chamber 3.

In the preferred embodiments of the device 1 shown in the illustrations, the channel 10 is arranged below the filtering element 8.

Advantageously, the inlet port 5 and the discharge port 7 face onto the inner chamber 3 and onto the outer chamber 4 respectively and are arranged on opposite sides with respect to the filtering element 8. The filtering element 8 is therefore substantially placed between the inlet port 5 and the discharge port 7. This particular conformation of the device 1 allows improving the fluid dynamics inside the filtering chamber 3,4, thus facilitating the removal of stagnant air, inasmuch as the direction of introduction of the blood to be filtered is in accord with the exit direction of the air from the device itself. The particular reciprocal arrangement of the inlet/outlet ports/mouths also allows achieving an efficient separation of the air from the blood.

Furthermore, the outlet port 6 of the filtered blood is arranged laterally with respect to the filtering element 8, i.e., outside the ideal extension of the filtering element itself along its longitudinal axis. More in detail, the body 2 defines a connection 16 for the exit of the blood communicating on one side with the outlet port 6 and on the opposite side with the outside.

Alternative embodiments cannot however be ruled out with a different arrangement of the inlet port 5 and outlet port 6.

As shown in the illustrations, the device 1 comprises at least one interruption element 11 for interrupting the flow of filtered blood arranged in the outer chamber 4 and positioned substantially facing at least onto the outlet port 6.

Such interruption element 11 is suitable for hindering the flow of filtered blood towards the outlet port 6, deviating it upwards as shown by the arrows 9, to avoid it coming out of the outlet port itself immediately after the filtering stage and thereby facilitating the separation of any air bubbles still in the filtered blood.

In the preferred embodiment shown in the illustrations, the interruption element 11 has a substantially annular shape to surround the lower base 8b of the filtering element 8 and has a variable height, the height of its portion arranged in correspondence to the outlet port 6 being greater than that of the diametrically opposite portion.

Four alternative embodiments of the device 1 are described here below.

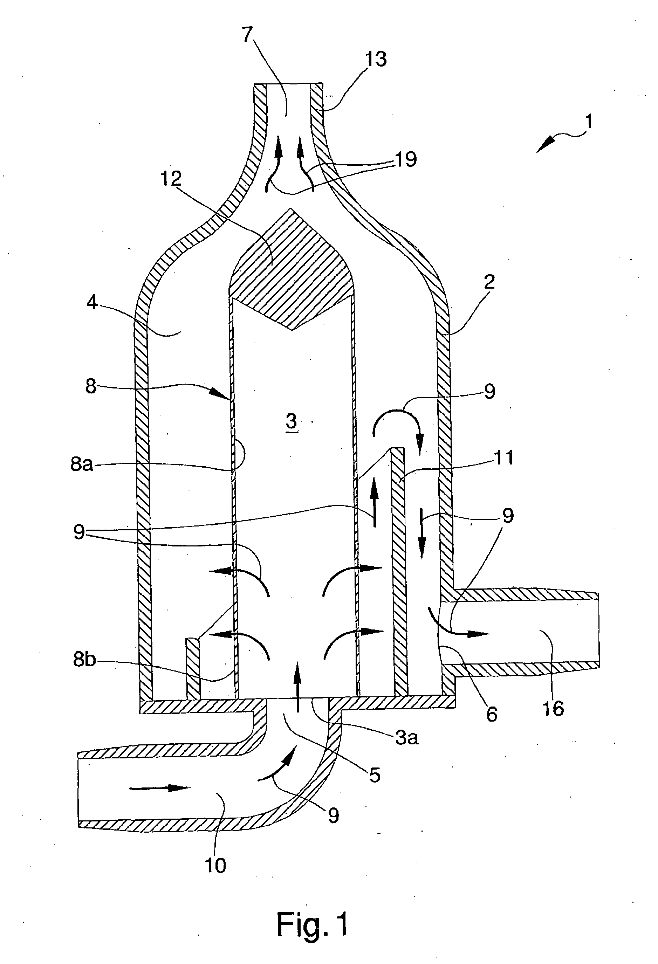

In the first embodiment illustrated in figure 1, the device 1 comprises an upper closing element 12 of the inner chamber 3.

Such closing element 12 is therefore associated with the filtering element 8 in correspondence to the upper base 8c thereof.

Suitably, in this embodiment, the surface of the closing element 12 facing onto the inner chamber 3 is substantially convex so as to push the air bubbles towards the filtering surface 8a.

The air still in the filtered blood, which collects up in the outer chamber 4 and that which passes through the filtering element 8 therefore exits through the discharge port 7.

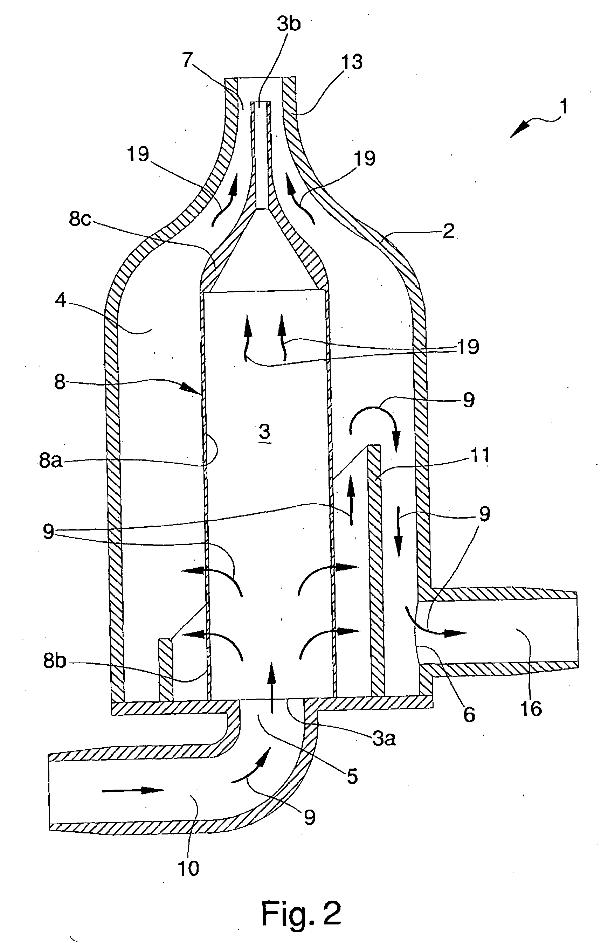

In the second embodiment shown in figure 2, the inner chamber 3 also has an outlet mouth 3b for the air which collects up inside it, where such outlet mouth 3b is defined in correspondence to the upper base 8c of the filtering element 8. Suitably, the body 2 has an entrance 13, defined in correspondence to the discharge port 7, and the outlet mouth 3b is arranged inside such entrance 13 to prevent the air coming out through it being able to enter the outer chamber 4 again and thus mix with the filtered blood.

The outlet mouth 3b is therefore contained inside the body 2 and arranged in communication with the outer chamber 4. Variations to this embodiment cannot however be ruled out which with the outlet mouth 3b being arranged outside the body 2.

Preferably, in this second embodiment, the outlet mouth 3b has a substantially capillary conformation, so as to prevent the air coming out of the outer chamber 4 through the discharge port 7 being able to enter the inner chamber 3.

The third embodiment of the device 1, not shown in detail in the illustrations, substantially has the same configuration as the second embodiment shown in figure 2 and, therefore, in this case as well, its inner chamber 3 has an outlet mouth 3b for the air.

Nevertheless, in this embodiment, the outlet mouth 3b has a larger cross section than that of the second embodiment described above and comprises a valve device (not shown), e.g., made up of a unidirectional membrane, suitable for allowing the air to come out of the inner chamber 3 and preventing it from coming in.

In the second and in the third embodiment described herein, the outlet mouth 3b and the discharge port 7 are substantially aligned with one another vertically. Furthermore, in these embodiments, the upper base 8b of the filtering element 8 is tapered towards the outlet mouth itself and is substantially closed laterally.

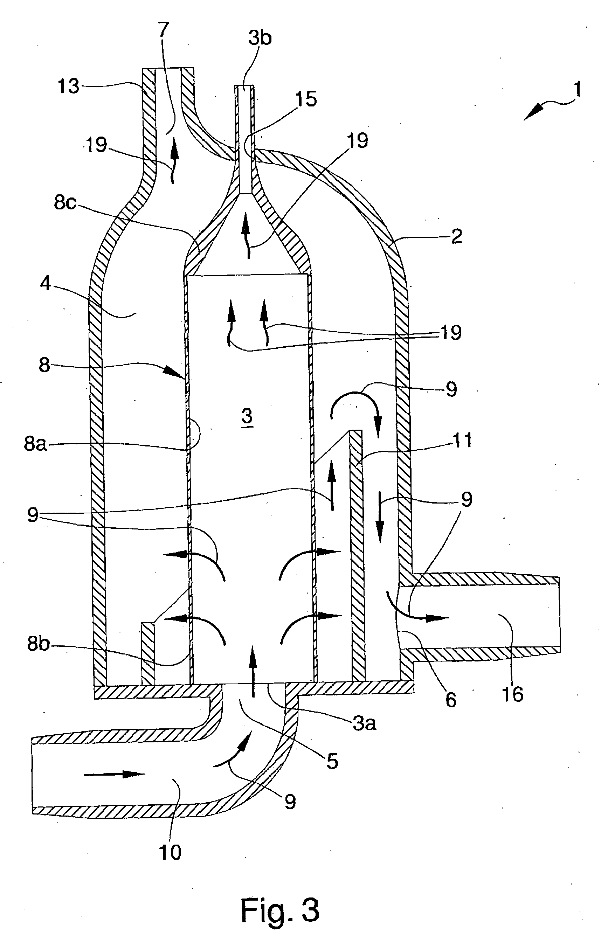

In the fourth embodiment shown in figure 3 as well, the inner chamber 3 has an outlet mouth 3b for the air contained inside it and defmed in correspondence to the upper base 8c of the filtering element 8.

In this fourth embodiment, the body 2 defines a further opening 15 separated from the discharge port 7 and the upper base 8c is associated with the body 2 in correspondence to such further opening 15.

More in particular, the body 2 and the upper base 8c are associated with one another so as to substantially separate the outlet mouth 3b from the outer chamber 4 and the air contained in the inner chamber 3 therefore flows outwards passing through the further opening 15.

This way, the discharge port 7 and the outlet mouth 3b are not in direct communication with one another and are suitable for conveying towards the outside of the body 2 air still in the filtered blood, and therefore contained in the outer chamber 4 and the air collected up in the inner chamber 3 respectively.

In the embodiment in figure 3, the upper base 8c fits through the further opening 15 and the outlet mouth 3b faces directly onto the outside of the body 2.

In a variation (not shown) of the fourth embodiment described herein, the upper base 8c is associated integral with the body 2 in correspondence to the further opening 15 and the outlet mouth 8b therefore remains inside the profile defined by the body itself.

In both these variations of the fourth embodiment, the air contained in the inner chamber 3 comes out towards the outside, passing through the further opening 15.

In this fourth embodiment as well, the upper base 8c is tapered towards the outlet mouth 3b and is substantially closed at the side.

The operation of the present invention is the following.

The blood to be filtered which enters the body 2 is conveyed by the channel 10 towards the inlet port 5 and enters the inner chamber 3 passing through the inlet mouth 3a. The blood to be filtered then enters the inner chamber 3 proceeding from the bottom upwards.

Once it has entered the inner chamber 3, the blood to be filtered passes through the filtering surface 8a, while the air bubbles collect up naturally, by effect of their lower weight, in the upper part of the inner chamber itself.

The air bubbles then move substantially along the entire length of the inner chamber 3, thereby favouring their detachment from the blood to be filtered. The blood to be filtered crossing the filtering element 8 collects up in the outer chamber 4 and impacts the interruption element 11 before coming out through the outlet port 6.

As can be seen by observing the arrows 9, the interruption element 11 causes a diversion upwards of the blood coming out of the filtering element 8 so as to make a further separation of any air bubbles still inside it.

Any air bubbles still inside the filtered blood collect up in the upper part of the outer chamber 4 and exit outside from the discharge port 7.

The way the air bubbles in the inner chamber 3 come out varies, on the other hand, according to the embodiment of the device 1.

More in particular, in the first embodiment described above, the air bubbles collected in the upper part of the inner chamber 3 are directed by the closing element 12 towards the filtering surface 8a, in such a way that, having crossed the filtering element 8, they are in the proximity of the discharge port 7 and do not mix again with the filtered blood which instead collects up in the lower part of the outer chamber 4.

In the second and in the third embodiment of the device 1, the air bubbles in the inner chamber 3 move naturally towards the outlet mouth 3b. The air bubbles which exit from the outlet mouth 3b find themselves inside the entrance 13 through which they exit outside the body 2. The presence of the entrance 13, which increases the exit speed of the bubbles towards the outside, facilitates the exit of the air bubbles arriving from the inner chamber 3 and reduces the risk of these passing into the outer chamber 4.

Furthermore, the capillarity of the outlet mouth 3b or the presence of the valve device prevents the air bubbles coming from the outer chamber 4 from entering the inner chamber 3.

In the fourth embodiment, on the other hand, the inner chamber 3 and the outer chamber 4 are substantially separate from one another, and so the air bubbles in the inner chamber 3 and in the outer chamber 4 exit outside the body 2 passing through the outlet mouth 3b and through the discharge port 7 respectively.

The device 1 can also be coupled in a monolithic way with an oxygenator device not shown in the illustrations.

The present invention therefore also concerns a method for filtering arterial blood by means of the device 1 described above and which comprises the following stages:
- introduction of the blood to be filtered into the inner chamber 3 through the lower base 8b of the filtering element 8, the air bubbles in the blood to be filtered collecting up in the upper part of the inner chamber 3;
- filtering of the blood contained in the inner chamber 3 through the filtering element 8, the filtered blood and the air bubbles collecting up in the outer chamber 4 and at least in the upper part of the inner chamber 3 respectively;
- exit of the filtered blood from the outer chamber 4 through the outlet port 6.

It has in fact been ascertained how the described invention achieves the proposed objects and in particular the fact is underlined that the introduction from below of the blood to be filtered inside the filtering device permits avoiding the formation of stagnant air inside the device.

The device according to the invention therefore allows avoiding the need to remove stagnant air manually, as in the case of devices of known type, thereby facilitating the integration thereof with a blood oxygenation device.

Again, the device according to the invention allows achieving more efficient removal of the air bubbles present in the blood coming from the oxygenator compared to known devices.

## Claims

1. Device (1) for filtering arterial blood comprising:
- at least a boxy body (2) which defines a filtering chamber (3, 4) having at least an inlet port (5) of the blood to be filtered, at least an outlet port (6) of the filtered blood and at least an air discharge port (7), the latter being defined in correspondence to the upper portion of the filtering chamber itself;
- at least an internally hollow filtering element (8) arranged at least in part inside said filtering chamber (3, 4), said body (2) and said filtering element (8) delimiting at least an inner chamber (3) and an outer chamber (4) with respect to the filtering element itself, wherein said outer chamber (4) surrounds said inner chamber (3);
where said inner and outer chambers (3, 4) communicate with said inlet port (5) and with said outlet port (6) respectively, and where said inner chamber (3) has at least an inlet mouth (3a) of the blood to be filtered defined in correspondence to the lower base (8b) of said filtering element (8), the blood to be filtered passing through said inlet mouth (3a), entering said inner chamber (3) and crossing said filtering element (8) to collect in said outer chamber (4), the air bubbles collecting in the upper part of the inner chamber itself,
**characterized by** the fact that it comprises at least an interruption element (11) of the flow of filtered blood arranged in said outer chamber (4) and positioned substantially facing at least onto said outlet port (6), said interruption element (11) being suitable for hindering the flow of the filtered blood towards said outlet port (6) deviating it upwards.

2. Device (1) according to claim 1, **characterised by** the fact that said body (2) defines at least a channel (10) for the flow of the blood to be filtered communicating with said inlet port (5) and suitable for introducing the blood to be filtered in said inner chamber (3) through said inlet mouth (3a), where the lower base (8b) of said filtering element (8) is arranged in proximity of said inlet port (5).

3. Device (1) according to claim 1 or 2, **characterised by** the fact that said channel (10) is suitable for conveying the blood to be filtered in correspondence to the median area of said inlet mouth (3a).

4. Device (1) according to one or more of the preceding claims, **characterised by** the fact that said inlet port (5) and said outlet port (6) are both arranged at the lower portion of said body (2).

5. Device (1) according to one or more of the preceding claims, **characterised by** the fact that said outlet port (6) is arranged on the side of said filtering element (8).

6. Device (1) according to one or more of the preceding claims, **characterised by** the fact that said inlet port (5) and said discharge port (7) face onto said inner chamber (3) and onto said outer chamber (4) respectively and are arranged on substantially opposite parts of said filtering element (8).

7. Device (1) according to one or more of the preceding claims, **characterised by** the fact that it comprises an upper closing element (12) of said inner chamber (3), the surface of said closing element (12) facing onto said inner chamber (3) being substantially convex to push the air bubbles towards said filtering element (8).

8. Device (1) according to one or more of the claims from 1 to 6, **characterised by** the fact that said inner chamber (3) has an air outlet mouth (3b) defined in correspondence to the upper base (8c) of said filtering element (8).

9. Device (1) according to claim 8, **characterised by** the fact that said body (2) has an entrance (13) defined in correspondence to said discharge port (7) and by the fact that said air outlet mouth (3b) is arranged substantially inside said entrance (13).

10. Device (1) according to claim 8 or 9, **characterised by** the fact that said outlet mouth (3b) has a substantially capillary conformation.

11. Device (1) according to claim 8 or 9, **characterised by** the fact that it comprises at least a valve element arranged in correspondence to said outlet mouth (3b) and suitable for allowing/preventing the exit/entry of air from/to said inner chamber (3).

12. Device (1) according to one or more of the claims from 8 to 11, **characterised by** the fact that said inlet mouth (3a), said outlet mouth (3b) and said air discharge port (7) are substantially aligned with one another.

13. Device (1) according to claim 8, **characterised by** the fact that said body (2) has a further opening (15) separated from said discharge port (7) and that the upper base (8c) of said filtering element (8) is associated with said body (2) in correspondence to said further opening (15) in such a way as to substantially separate said outlet mouth (3b) from said outer chamber (4).

14. Equipment for oxygenating and filtering blood, **characterised by** the fact that it comprises at least a filtering device (1) according to one or more of the preceding claims and a device for blood oxygenation associated integrally with each other.

## Patentansprüche

1. Vorrichtung (1) zum Filtern von arteriellem Blut, mit:
- wenigstens einem kastenförmigen Gehäuse (2), welches eine Filterkammer (3, 4) bildet, die wenigstens einen Einlassanschluss (5) für zu filterndes Bluts, wenigstens einen Auslassanschluss (6) für das gefilterte Blut und wenigstens einen Luftaustrittsanschluss (7) aufweist, wobei der letztere in Zusammenspiel mit dem oberen Bereich der Filterkammer ausgebildet ist;
- wenigstens einem inneren hohlen Filterelement (8), das wenigstens teilweise innerhalb der Filterkammer (3, 4) angeordnet ist, wobei das Gehäuse (2) und das Filterelement (8) wenigstens eine Innenkammer (3) und eine Außenkammer (4) in Bezug zu dem Filterelement begrenzen, wobei die Außenkammer (4) die Innenkammer (3) umgibt;
wobei die Innen-und die Außenkammer (3, 4) jeweils mit dem Einlassanschluss (5) und mit dem Auslassanschluss (6) kommunizieren und wobei die Innenkammer (3) wenigstens eine Einlassöffnung (3a) für das zu filternde Blut hat, die in Zusammenspiel mit der unteren Basis (8b) des Filterelements (8) ausgebildet ist, wobei das zu filternde Blut durch die Einlassöffnung (3a) hindurchgeht, in die Innenkammer (3) eintritt und das Filterelement (8) durchquert, um sich in der Außenkammer (4) zu sammeln, wobei sich die Luftblasen in dem oberen Teil der Innenkammer sammeln, **dadurch gekennzeichnet, dass** diese wenigstens ein Unterbrechungselement (11) für den Fluss des gefilterten Bluts umfasst, das in der Außenkammer (4) angeordnet und im Wesentlichen wenigstens dem Auslassanschluss (6) zugewandt positioniert ist, wobei das Unterbrechungselement (11) dazu geeignet ist, den Fluss des gefilterten Bluts in Richtung des Auslassanschlusses (6) daran zu hindern, in Richtung des Auslassanschlusses (6) nach oben abgelenkt zu werden.

2. Vorrichtung (1) nach Anspruch 1, **dadurch gekennzeichnet, dass** das Gehäuse (2) wenigstens einen Kanal (10) für den Fluss des zu filternden Bluts bildet, der mit dem Einlassanschluss (5) kommuniziert und dazu geeignet ist, dass zu filternde Blut durch die Einlassöffnung (3a) hindurch in die Innenkammer (3) einzuleiten, wobei die untere Basis (8b) des Filterelements (8) in der Nähe des Einlassanschlusses (5) angeordnet ist.

3. Vorrichtung (1) nach Anspruch 1 oder 2, **dadurch gekennzeichnet, dass** der Kanal (10) dazu geeignet ist, das zu filternde Blut in Zusammenspiel mit dem mittleren Bereich der Einlassöffnung (3a) zu fördern.

4. Vorrichtung (1) nach ein oder mehreren der vorstehenden Ansprüche, **dadurch gekennzeichnet, dass** der Einlassanschluss (5) und der Auslassanschluss (6) beide am unteren Bereich des Gehäuses (2) angeordnet sind.

5. Vorrichtung (1) nach ein oder mehreren der vorstehenden Ansprüche, **dadurch gekennzeichnet, dass** der Auslassanschluss (6) auf der Seite des Filterelements (8) angeordnet ist.

6. Vorrichtung (1) nach ein oder mehreren der vorstehenden Ansprüche, **dadurch gekennzeichnet, dass** der Einlassanschluss (5) und der Auslassanschluss (7) jeweils der Innenkammer (3) und der Außenkammer (4) zugewandt sind und an den im wesentlichen entgegengesetzten Teilen des Filterelements (8) angeordnet sind.

7. Vorrichtung (1) nach ein oder mehreren der vorstehenden Ansprüche, **dadurch gekennzeichnet, dass** diese ein oberes Schließelement (12) für die Innenkammer (3) umfasst, wobei die Oberfläche des Schließelements (12), welche der Innenkammer (3) zugewandt ist, im Wesentlichen konvex ist, um die Luftblasen in Richtung des Filterelements (8) zu drücken.

8. Vorrichtung (1) nach ein oder mehreren der Ansprüche 1 bis 6, **dadurch gekennzeichnet, dass** die Innenkammer (3) eine Luftauslassöffnung (3b) hat, die im Zusammenspiel mit der oberen Basis (8c) des Filterelements (8) ausgebildet ist.

9. Vorrichtung (1) nach Anspruch 8, **dadurch gekennzeichnet, dass** das Gehäuse (2) einen Eingang (13) hat, der im Zusammenspiel mit dem Austrittsanschluss (7) ausgebildet ist, und dadurch, dass die Luftauslassöffnung (3b) im Wesentlichen innerhalb des Eingangs (13) angeordnet ist.

10. Vorrichtung (1) nach Anspruch 8 oder 9, **dadurch gekennzeichnet, dass** die Auslassöffnung (3b) eine im Wesentlichen kapillare Gestaltung hat.

11. Vorrichtung (1) nach Anspruch 8 oder 9, **dadurch gekennzeichnet, dass** diese wenigstens ein Ventilelement umfasst, das im Zusammenspiel mit der Auslassöffnung (3b) angeordnet ist und dazu geeignet ist, den Austritt/Eintritt von Luft aus/ in die Innenkammer (3) zu erlauben/zu verhindern.

12. Vorrichtung (1) nach ein oder mehreren der Ansprüche 8 bis 11, **dadurch gekennzeichnet, dass** die Einlassöffnung (3a), die Auslassöffnung (3b) und der Luftaustrittsanschluss (7) im Wesentlichen zueinander ausgerichtet sind.

13. Vorrichtung (1) nach Anspruch 8, **dadurch gekennzeichnet, dass** das Gehäuse (2) eine weitere Öffnung (15) hat, die von dem Austrittsanschluss (7) abgetrennt ist, und dass die obere Basis (8c) des Filterelements (8) mit dem Gehäuse (2) im Zusammenspiel mit der weiteren Öffnung (15) verbunden ist, in der Weise, dass die Auslassöffnung (3b) von der Außenkammer (4) im Wesentlichen abgetrennt ist.

14. Apparatur zum Oxigenieren und Filtern von Blut, **dadurch gekennzeichnet, dass** diese wenigstens eine Filtervorrichtung (1) gemäß ein oder mehreren der vorstehenden Ansprüche und eine Vorrichtung zum Oxigenieren von Blut umfasst, die miteinander integriert verbunden sind.

## Revendications

1. Dispositif (1) de filtration de sang artériel comprenant :
- au moins un corps en forme de boîte (2) qui délimite une chambre de filtration (3, 4) équipée d'au moins un orifice d'entrée (5) du sang à filtrer, au moins un orifice de sortie (6) du sang filtré et au moins un orifice d'évacuation de l'air (7), ce dernier étant défini en correspondence avec la portion supérieure de la chambre de filtration proprement dite ;
- au moins un élément filtrant (8) creux intérieurement agencé au moins partiellement à l'intérieur de ladite chambre de filtration (3, 4), ledit corps (2) et ledit élément filtrant (8) délimitant au moins une chambre intérieure (3) et une chambre extérieure (4) par rapport à l'élément filtrant proprement dit, dans lequel ladite chambre extérieure (4) entoure ladite chambre intérieure (3) ;
dans lequel lesdites chambres intérieure et extérieure (3, 4) communiquent avec ledit orifice d'entrée (5) et avec ledit orifice de sortie (6) respectivement, and dans lequel ladite chambre intérieure (3) dispose d'au moins une bouche d'entrée (3a) du sang à filtrer définie en correspondence avec la base inférieure (8b) dudit élément filtrant (8), le sang à filtrer passant par ladite bouche d'entrée (3a), entrant dans ladite chambre intérieure (3) et traversant ledit élément filtrant (8) pour se collecter dans ladite chambre extérieure (4), les bulles d'air se collectant dans la partie supérieure de la chambre intérieure proprement dite,
**caractérisé en ce qu'**il comprend au moins un élément d'interruption (11) de l'écoulement du sang filtré agencé dans ladite chambre extérieure (4) et positionné sensiblement en regard au moins dudit orifice de sortie (6), ledit élément d'interruption (11) étant apte à faire obstacle à l'écoulement du sang filtré vers ledit orifice de sortie (6) en le déviant vers le haut.

2. Dispositif (1) selon la revendication 1, **caractérisé en ce que** ledit corps (2) définit au moins un canal (10) pour l'écoulement du sang à filtrer en communication avec ledit orifice d'entrée (5) et apte à introduire le sang à filtrer dans ladite chambre intérieure (3) à travers ladite bouche d'entrée (3a), dans lequel la base inférieure (8b) dudit élément filtrant (8) est agencée à proximité dudit orifice d'entrée (5).

3. Dispositif (1) selon la revendication 1 ou 2, **caractérisé en ce que** ledit canal (10) est apte à acheminer le sang à filtrer en correspondence avec la zone médiane de ladite bouche d'entrée (3a).

4. Dispositif (1) selon l'une ou plusieurs des revendications précédentes, **caractérisé en ce que** ledit orifice d'entrée (5) et ledit orifice de sortie (6) sont tous les deux agencés au niveau de la portion inférieure dudit corps (2).

5. Dispositif (1) selon l'une ou plusieurs des revendications précédentes, **caractérisé en ce que** ledit orifice de sortie (6) est agencé à côté dudit élément filtrant (8).

6. Dispositif (1) selon l'une ou plusieurs des revendications précédentes, **caractérisé en ce que** ledit orifice d'entrée (5) et ledit orifice d'évacuation (7) donnent sur ladite chambre intérieure (3) et sur ladite chambre extérieure (4) respectivement et sont agencés sur des parties sensiblement opposées dudit élément filtrant (8).

7. Dispositif (1) selon l'une ou plusieurs des revendications précédentes, **caractérisé en ce qu'**il comprend un élément d'obturation supérieur (12) de ladite chambre intérieure (3), la surface dudit élément d'obturation (12) en regard de ladite chambre intérieure (3) étant sensiblement convexe pour pousser les bulles d'air vers ledit élément filtrant (8).

8. Dispositif (1) selon l'une ou plusieurs des revendications de 1 à 6, **caractérisé en ce que** ladite chambre intérieure (3) est munie d'une bouche de sortie d'air (3b) définie en correspondence avec la base supérieure (8c) dudit élément filtrant (8).

9. Dispositif (1) selon la revendication 8, **caractérisé en ce que** ledit corps (2) dispose d'une entrée (13) définie en correspondence avec ledit orifice d'évacuation (7) et **en ce que** ladite bouche de sortie d'air (3b) est agencée sensiblement à l'intérieur de ladite entrée (13).

10. Dispositif (1) selon la revendication 8 ou 9, **caractérisé en ce que** ladite bouche de sortie (3b) présente une conformation sensiblement capillaire.

11. Dispositif (1) selon la revendication 8 ou 9, **caractérisé en ce qu'**il comprend au moins un élément de valve agencé en correspondence avec ladite bouche de sortie (3b) et apte à permettre/empêcher la sortie/entrée d'air en provenance de/vers ladite chambre intérieure (3).

12. Dispositif (1) selon l'une ou plusieurs des revendications de 8 à 11, **caractérisé en ce que** ladite bouche d'entrée (3a), ladite bouche de sortie (3b) et ledit orifice d'évacuation de l'air (7) sont sensiblement alignés les uns avec les autres.

13. Dispositif (1) selon la revendication 8, **caractérisé en ce que** ledit corps (2) possède une autre ouverture (15) distincte dudit orifice d'évacuation (7) et que la base supérieure (8c) dudit élément filtrant (8) est associée audit corps (2) en correspondence avec ladite autre ouverture (15) de manière à sensiblement séparer ladite bouche de sortie (3b) de ladite chambre extérieure (4).

14. Équipement d'oxygénation et de filtration de sang, **caractérisé en ce qu'**il comprend au moins un dispositif de filtration (1) selon l'une ou plusieurs des revendications précédentes et un dispositif d'oxygénation du sang intégralement associés l'un à l'autre.
